# EUROPÄISCHE PATENTSCHRIFT

(11) **EP 0 511 233 B1**
(45) Veröffentlichungstag und Bekanntmachung des Hinweises auf die Patenterteilung: **08.03.1995**
(21) Anmeldenummer: 91901734.3
(22) Anmeldetag: 10.01.1991
(51) Int. Cl.: C07C 45/58, C07C 67/313, C07C 49/04, C07C 69/716

(54) **VERFAHREN ZUR HERSTELLUNG VON KETONVERBINDUNGEN**
METHOD OF PREPARATION OF KETONES
PROCEDE DE PREPARATION DE COMPOSES DE CETONES

(30) Priorität: 18.01.1990 DE 4001316
(43) Veröffentlichungstag der Anmeldung: 04.11.1992
(73) Patentinhaber: Henkel Kommanditgesellschaft auf Aktien, 40191 Düsseldorf (DE)
(72) Erfinder: STOLL, Gerhard , Dr., W-4052 Korschenbroich 1 (DE); GRUNDT, Elke, D-4010 Hilden (DE)
(86) Internationale Anmeldenummer: EP9100025
(87) Internationale Veröffentlichungsnummer: WO9110636

(56) Entgegenhaltungen:
- EP-A- 0 220 792
- DE-A- 3 334 600
- CHEMICAL COMMUNICATIONS, Band 4, 21. Februar 1968, S. 227-229; D. BETHELL et al: "Isomerisation of epoxides to carbonyl compounds induced by iodides in dimethyl sulphoxide"

## Beschreibung

Gegenstand der Erfindung ist ein Verfahren zur Herstellung von Ketonverbindungen durch Umlagerung von Epoxiden in Gegenwart von Iodidionen, bei dem als Umlagerungskatalysatoren quartäre Ammonium- oder Phosphoniumsalze, gegebenenfalls im Gemisch mit Alkali- oder Erdalkaliiodiden verwendet werden.

Ketonverbindungen mit langen, gegebenenfalls substituierten Alkyl- oder Arylresten stellen wertvolle Zwischenprodukte für die chemische Industrie dar und finden beispielsweise Verwendung als Antischaummittel oder bei der Herstellung von PVC-Costabilisatoren.

Zur Herstellung dieser Stoffe sind zwar eine Vielzahl von Methoden bekannt, die jedoch alle den Nachteil eines hohen präparativen Aufwandes aufweisen. So lassen sich Fettketone z. B. durch Pyrolyse von Magnesiumsalzen bei hohen Temperaturen [Organic Synthesis, **33**, 84] oder durch Umlagerung von Epoxiden in Gegenwart von Magnesiumhalogenid-Etherat erhalten [J.Am.Chem.Soc., **77**, 3070, 5083 (1955), J.Am.Oil.Chem.Soc., **79**, 6283 (1957)].

In der deutschen Patentanmeldung DE 36 01 380 A1 wird ein einfacheres Verfahren beschrieben, bei dem epoxidierte Fettsäureester in Gegenwart von Natriumiodid und Polyethylenglycolen bei 160 bis 200°C in die entsprechenden Oxoverbindungen umgelagert werden. Von Nachteil ist hierbei jedoch, daß ein Lösungsvermittler erforderlich ist und die Umlagerung nur mit geringen Ausbeuten erfolgt.

Der Erfindung lag die Aufgabe zugrunde, ein Verfahren zur Herstellung von Ketonverbindungen zu entwicklen, das frei von den beschriebenen Nachteilen ist.

Gegenstand der Erfindung ist ein Verfahren zur Herstellung von Ketonverbindungen durch Umlagerung von Epoxiden, die mindestens eine Oxirangruppe und mindestens sechs Kohlenstoffatome enthalten, wobei mindestens einer der vier Substituenten der Oxirangruppe ein Wasserstoffatom darstellt, in Gegenwart von Iodidionen, bei dem als Umlagerungskatalysatoren quartäre Ammonium- oder Phosphoniumsalze, gegebenenfalls im Gemisch mit Alkali- oder Erdalkaliiodiden, verwendet werden.

Auf diese Weise lassen sich Epoxide ohne Lösungsvermittler mit hohen Ausbeuten in die entsprechenden Ketonverbindungen überführen.

Stoffe, die im Rahmen der Erfindung in Ketonverbindungen umgelagert werden können, umfassen folgende Verbindungsklassen:
a) Epoxide aliphatischer oder cycloaliphatischer Monoolefine mit 6 bis 30 Kohlenstoffatomen, wie z. B. Hexen-1, Cyclohexen, Octen-1, Cycloocten, Decen-1, Dodecen-1, Cyclododecen, Tetradecen-1, Hexadecen-1, Octadecen-1, Octen-2, Octen-3, Octen-4, Decen-5, Dodecen-6, Tetradecen-7 oder Octadecen-9. Vorzugsweise geht man von Olefinen mit 12 bis 18 Kohlenstoffatomen aus.
b) Epoxide von Estern ungesättigter Fettsäuren mit 11 bis 22 Kohlenstoffatomen und 1, 2 oder 3 Doppelbindungen mit linearen oder verzweigten, aliphatischen, gesättigten oder ungesättigten Alkoholen mit 1 bis 22 Kohlenstoffatomen und 0, 1, 2 oder 3 Doppelbindungen, araliphatischen Alkoholen mit 7 bis 15 Kohlenstoffatomen oder Phenolen.
   Beispiele typischer ungesättigter Fettsäuren sind Undecylensäure, Palmitoleinsäure, Elaidinsäure, Linolsäure, Linolensäure, Chaulmograasäure oder Erucasäure. Vorzugsweise geht man von Ölsäure- oder Petroselinsäureestern aus.
   Beispiele typischer gesättigter Alkohole sind Ethanol, Propanol-1, Propanol-2, Capronalkohol, Caprylalkohol, Caprinalkohol, Laurylalkohol, Myristylalkohol, Cetylalkohol, Stearylalkohol oder Behenylalkohol. Vorzugsweise geht man von Alkoholen mit 1 bis 8 Kohlenstoffatomen aus, unter denen die Verwendung von Methyl-, Butyl und 2-ethylhexylestern besonders bevorzugt ist.
   Beispiele typischer ungesättigter Alkohole sind Allylalkohol, Undecenylalkohol, Palmitoleylalkohol, Elaidylalkohol, Linoleylalkohol, Linolenylalkohol oder Erucylalkohol. Vorzugsweise geht man Oleyl- oder Petroselinylestern aus.
   Ester ungesättigter Fettsäuren mit ungesättigten Fettalkoholen können die Oxirangruppe in der Fettsäurekomponente und/oder der Alkoholkomponente enthalten. Sind Fettsäure- und/oder Alkoholkomponente mehrfach ungesättigt, können sie auch mehr als eine Oxirangruppe aufweisen.
c) Epoxide vom Estern ungesättigter Fettsäuren mit Polyolen, z. B. Ethylenglycol, Diethylenglycol, Triethylenglycol, Propylenglycol, Diglycerin, Triglycerin, Trimethylolpropan, Pentaerythrit oder Sorbitan. Vorzugsweise wird jedoch von ungesättigten Fettsäureglycerinestern ausgegangen.
   Unter Fettsäureglycerinester sind dabei die Mono-, Di- und Triester sowie deren Gemische zu verstehen, wie sie z. B. bei der Herstellung durch Veresterung von 1 Mol Glycerin mit 1 bis 3 Mol ungesättigter Fettsäure oder bei der Umesterung von ungesättigten Triglyceriden mit 0.5 bis 2 Mol Glycerin erhalten werden. Insbesondere werden jene Epoxide ungesättigter Fettsäureglycerinester umgelagert, die sich von Fettsäuren mit 16 bis 24 Kohlenstoffatomen und 1 bis 5 Doppelbindungen ableiten, so z. B. Palmitoleinsäure, Ölsäure, Elaidinsäure, Petroselinsäure, Erucasäure, Linolsäure, Linolensäure, Chaulmograasäure, Arachidonsäure oder Clupanodonsäure. Aus Gründen der leichten Zugänglichkeit wird bevorzugt von technischen Fettsäureglycerinestergemischen ausgegangen, deren Fettsäurekomponente mehr als 50 Gew.-% Ölsäure oder Linolsäure enthält.
   Die Fettsäureglycerinester können synthetischer oder natürlicher Herkunft sein. Vorzugsweise werden solche Ester verwendet, die aus Sojaöl, Baumwollsaatöl, Erdnußöl, Olivenöl, Leinöl, Lardöl, Meadowfoamöl, Chaulmograaöl, Schweineschmalz oder Fischöl gewonnen werden, wobei ölsäurereiches Rüböl, ölsäurereiches Sonnenblumenöl oder Korianderöl besonders bevorzugt sind. Ausdrücklich können auch solche nativen Fettsäureglycerinester eingesetzt werden, deren Fettsäurekomponente sich nicht vollständig, sondern nur zum überwiegenden Teil, d. h. zu mehr als 50 Gew.-% aus den genannten ungesättigten Fettsäuren aufbaut sowie technische Gemische verschiedener ungesättigter oder weitgehend ungesättigter Fettsäureglycerinester untereinander, sofern der Gehalt an ungesättigten Fettsäuren in den Mischungen wiederum mehr als 50 Gew.-% beträgt.
d) Epoxide von Estern gesättigter aliphatischer Carbonsäuren mit 1 bis 22 Kohlenstoffatomen mit aliphatischen ungesättigten Alkoholen mit 1 bis 22 Kohlenstoffatomen und 1, 2 oder 3 Doppelbindungen.
   Beispiele typischer gesättigter aliphatischer Carbonsäuren sind Ameisensäure, Propionsäure, Buttersäure, Valeriansäure, Capronsäure, Caprylsäure, Caprinsäure, Laurinsäure, Myristinsäure, Palmitinsäure oder Behensäure. Vorzugsweise geht man von Essigsäure- oder Stearinsäureestern aus.
   Beispiele typischer ungesättigter Alkohole sind Allylalkohol Undecenylalkohol, Palmitoleylalkohol, Elaidylalkohol, Linoleylalkohol, Linolenylalkohol oder Erucylalkohol. Vorzugsweise geht man von Oleyl- oder Petroselinylestern aus.
e) Epoxide von Alkenylethern und Alkenyl(poly)alkylenglycolethern, die einen Alkenylrest mit 11 bis 22 Kohlenstoffatomen und 1, 2 oder 3 Doppelbindungen und einen weiteren linearen oder verzweigten Alkyl- oder Alkenylrest mit 1 bis 22 Kohlenstoffatomen, einen Aralkylrest mit 7 bis 15 Kohlenstoffatomen oder einen Phenylrest aufweisen. Die Polyalkylenglycolether können Polyethylenglycol- oder Polypropylenglycolether darstellen und 1 bis 10, vorzugsweise 1 bis 5 Alkylenglycoleinheiten enthalten.
   Alkenylether dieser Art sind z. B. nach der Williamson'schen Ethersynthese erhältlich, bei der man einen ungesättigten Fettalkohol oder dessen Alkylenoxidanlagerungsprodukt mit einem entsprechenden Alkyl-, Alkenyl-, Aralkyl- oder Arylhalogenid umsetzt.
   Typische Beispiele für Alkenylether sind Diundecenylether, Dioleylether, Dielaidylether, Dipetroselinylether, Oleylmethylether, Oleylbutylether, Oleylbenzylether, Oleylalkohol-2-EO-methylether, Oleylalkohol-4-EO-butylether oder Oleylalkohol-5-EO-benzylether.

Epoxide der genannten Art werden durch Epoxidation ungesättigter Verbindungen, beispielsweise nach dem in der Patentschrift DE 857 364 beschriebenen Verfahren durch Umsetzung mit Peressigsäure in Anwesenheit saurer Katalysatoren oder mit in-situ aus Ameisensäure und Wasserstoff gebildeter Perameisensäure gemäß der Patentschrift US 24 85 160 erhalten.

In den als Umlagerungskatalysatoren verwendeten quartären Ammonium- oder Phosphoniumsalzen kommen als Substituenten lineare und/oder verzweigte Alkylreste mit 1 bis 22 Kohlenstoffatomen, wie z. B. Ethyl-, Propyl-, 2-Ethylhexyl-, Lauryl- oder Cetylreste, Aralkylreste mit 7 bis 15 Kohlenstoffatomen, wie z. B. Benzylreste sowie Arylreste mit 6 bis 12 Kohlenstoffatomen in Betracht. Vorzugsweise werden methyl-, butyl-, phenyl- und/oder stearylsubstituierte Ammonium- oder Phosphoniumsalze als Umlagerungskatalysatoren eingesetzt.

Die quartärem Ammonium- oder Phosphoniumsalze können die Alkyl-, Aralkyl- oder Arylreste in beliebigem Substitutionsmuster enthalten. Es hat sich jedoch als besonders vorteilhaft erwiesen, Ammonium- oder Phosphoniumsalze mit vier Substituenten gleicher Art oder zwei kurzkettigen und zwei längerkettigen Substituenten einzusetzen. Beispiele hierfür sind Tetrabutylphosphonium- oder Dimethyldistearylammoniumsalze. Sie können entweder als solche eingesetzt werden oder aus tert.-Aminen bzw. Phosphinen und Alkyl- oder Arylhalogeniden in situ erzeugt werden.

Die quartären Ammonium- und Phosphoniumsalze können als Halogenide oder Pseudohalogenide vorliegen. Insbesondere kommen Chloride und bevorzugt Iodide in Betracht.

Infolge ihrer leichten Zugänglichkeit, hohen katalytischen Aktivität, chemischen Stabilität und problemlosen Abtrennbarkeit ist die Verwendung von Tetrabutyl- und Tetraphenylphosphoniumiodid zur Umlagerung von Epoxiden in Ketonverbindungen besonders bevorzugt.

Die Konzentration der quartären Ammonium- oder Phosphoniumsalze kann 0,05 bis 10 Mol-% bezogen auf das Epoxid betragen. Ausbeuten von mehr als 70 % bezogen auf das eingesetzte Epoxid und kurze Reaktionszeiten werden jedoch erst bei Einsatzkonzentrationen oberhalb von 0.5 Mol-% erhalten, während Einsatzmengen von mehr als 5 Mol-% die Reaktionszeiten nur noch unwesentlich verkürzen.

In einer bevorzugten Ausführungsform der Erfindung werden die quartären Ammonium- oder Phosphoniumsalze in Form ihrer Iodide eingesetzt. In Gegenwart von Alkali- oder Erdalkaliiodiden, insbesondere Natriumiodid, können jedoch auch die entsprechenden Chloride oder Bromide verwendet werden.

Die Einsatzkonzentration des Alkali- oder Erdalkaliiodids kann bezogen auf das Epoxid 0.1 bis 8 Mol-%, vorzugsweise 0.1 bis 5 Mol-% betragen, während das molare Verhältnis von quartärem Ammonium- oder Phosphoniumsalz zu Alkali- oder Erdalkaliiodid bei 2 : 1 bis 1 : 5 liegt.

Aus Gründen der leichten Verfügbarkeit und hohen Aktivität werden in einer weiteren bevorzugten Ausführungsform der Erfindung Gemische von Dimethyldistearylammoniumchlorid und Natriumiodid verwendet.

Zur Umlagerung der Epoxide in Ketonverbindungen werden Ausgangsstoffe und Katalysator bzw. Katalysatormischung unter Inertgas bei 160 bis 230, vorzugsweise 180 bis 200°C, 120 bis 600 min gerührt. Sind die durch Umlagerung erhaltenen Ketonverbindungen destillierbar, so kann das Umlagerungsprodukt durch Destillation, gegebenenfalls im Vakuum gewonnen werden. Der das Ammonium- oder Phosphoniumsalz und gegebenenfalls Natriumiodid enthaltende Destillationsrückstand ist katalytisch aktiv und kann in diesen Fällen ohne Aufarbeitung in die Umlagerung zurückgeführt werden. Kann das Umlagerungsprodukt nicht destilliert werden, genügt es, den Katalysator durch Auswaschen mit heißem Wasser abzutrennen.

In vielen Fällen ist es nicht erforderlich, das Epoxid vollständig in die entsprechende Ketonverbindungen umzulagern. Es kann z. B. ausreichen, Gemische herzustellen, die bezogen auf das Epoxid nur 20 bis 50 Gew.-% des Umlagerungsproduktes enthalten. Derartige technische Gemische lassen sich unter laufender Kontrolle des Epoxidsauerstoffgehaltes des Reaktionsgemisches bis zum Erreichen des gewünschten Restgehaltes an Epoxidsauerstoff herstellen.

Die folgenden Beispiele sollen den Gegenstand der Erfindung näher erläutern.

### Beispiele

### Einsatzstoffe (Edukte, E)

E1

| Epoxidierter technischer Ölsäuremethylester | |
|---|---|
| Epoxidsauerstoffgehalt (EpOZ) | 4.95 Gew.-% |
| Gehalt 9,10-Epoxystearinsäuremethylester | 70 Gew.-% |
| Verseifungszahl (VZ) | 187.8 |

E2

| Epoxidiertes n-C_{13/14}-Olefingemisch | |
|---|---|
| Epoxidsauerstoffgehalt (EpOZ) | 6.74 Gew.-% |
| Gehalt epoxidiertes n-C₁₃-Olefin | 58 Gew.-% |
| Gehalt epoxidiertes n-C₁₄-Olefin | 40 Gew.-% |

E3

| Epoxidiertes ölsäurereiches Sonnenblumenöl | |
|---|---|
| Epoxidsauerstoffgehalt (EpOZ) | 4.55 Gew.-% |
| Ölsäuregehalt vor Epoxidation | 80 Gew.-% |
| Verseifungszahl (VZ) | 183.0 |
| Iodzahl (IZ) | 2.1 |
| Säurezahl (SZ) | 3.4 |

E4

| Epoxidiertes Sojaöl | |
|---|---|
| Epoxidsauerstoffgehalt (EpOZ) | 6.78 Gew.-% |
| Verseifungszahl (VZ) | 181.8 |
| Iodzahl (IZ) | 3.4 |
| Säurezahl (SZ) | 0.4 |

E5

| 1,2-Epoxyoctadecan | |
|---|---|
| Epoxidsauerstoffgehalt (EpOZ) | 5.42 Gew.-% |

### Tetrasubstituierte Ammonium- bzw. Phosphoniumsalze (A)

- A1: Tetrabutylphosphoniumiodid
M = 386, Fa.Aldrich
- A2: Tetrabutylphosphoniumbromid
M = 339, Fa.Aldrich
- A3: Dimethyldistearylammoniumchlorid
M = 588, Dehyquart^{R)} DAM, Fa.Henkel

### Umlagerung von Epoxiden:

### Beispiel 1 - 5:

**Allgemeine Vorschrift zur Umlagerung von epoxidiertem Ölsauremethylester**. In einem Dreihalskolben mit Rührer, Rückflußkühler und Innenthermometer wurden 500 g (1.55 Mol) epoxidierter Ölsäuremethylester (E1) vorgelegt und mit 1 bis 5 Mol-% (bezogen auf das Epoxid) eines Tetraalkylammonium- oder -phosphoniumsalzes (A1, A2, A3) und gegebenenfalls mit 1 bis 5 Mol-% (bezogen auf das Epoxid) Natriumiodid versetzt. Die Reaktionsmischung wurde über einen Zeitraum t = 165 bis 585 min und bei einer Temperatur T = 180 bis 200°C unter Stickstoff gerührt. Durch Destillation im Vakuum (0.04 bis 0.02 mbar, 200°C Sumpftemperatur) wurde als Umlagerungsprodukt ein Gemisch aus 9- und 10-Ketostearinsäuremethyester als gelber kristalliner Feststoff sowie ein Destillationsrückstand gewonnen, der den Umesterungskatalysator enthielt. Die Kenndaten der Produkte befinden sich in Tab.1.

### Beispiel 6:

Beispiel 1 wurde wiederholt. Als Katalysator wurde der Destillationsrückstand (112.8 g) aus Beispiel 1 eingesetzt. Kenndaten des Produktes befinden sich in Tab.1.

### Beispiel 7:

**Umlagerung von epoxidierten n-Olefinen**. In einem Dreihalskolben mit Rührer, Rückflußkühler und Innenthermometer wurden 500 g (2.1) Mol einer epoxidierten Mischung aus n-Olefinen (E2) vorgelegt und mit 2 Mol-% (bezogen auf das Epoxid) Tetrabutylphosphoniumbromid (A1) und 1 Mol-% (bezogen auf das Epoxid) Natriumiodid versetzt. Die Reaktionsmischung wurde unter Stickstoff 420 min bei 180°C gerührt. Nach Abkühlen und zweimaligem Waschen mit je 250 ml 30°C warmen Wassers wurde die organische Phase im Vakuum (0.04 bis 0.02 mbar, Sumpftemperatur 80 bis 130°C) abdestilliert. Das Umlagerungsprodukt wurde als hellgelbe, blanke Flüssigkeit erhalten. Kenndaten des Produktes befinden sich in Tab.1.

### Beispiel 8 - 10:

**Partielle Umlagerung von epoxidierten Fettsäureglycerinestern.** In einem Dreihalskolben mit Rührer, Rückflußkühler und Innenthermometer wurden 1000 g (2.81 Mol) eines epoxidierten Fettsäureglycerinesters (E3, E4) vorgelegt und mit 0.64 bis 1.2 Mol-% (bezogen auf das Epoxid) eines Tetraalkylammonium- oder -phosphoniumsalzes (A1, A3) und gegebenenfalls 2.3 Mol-% (bezogen auf das Epoxid) Natriumiodid versetzt. Die Reaktionsmischung wurde unter Stickstoff über einen Zeitraum von t = 270 bis 540 min bei einer Temperatur von T = 200°C gerührt. Nach Abkühlen und viermaligem Waschen mit 60°C heißem Wasser und Trocknen des Produktes im Vakuum (0.02 mbar, 90°C) wurden die partiellen Umlagerungsprodukte als braune Feststoffe erhalten. Kenndaten der Produkte befinden sich in Tab.1.

### Beispiel 11:

**Umlagerung von 1.2-Epoxyoctadecan**. In einem Dreihalskolben mit Rührer, Rückflußkühler und Innenthermometer wurden 1000 g (3.4 Mol) 1.2-Epoxyoctadecan (E5) vorgelegt und mit 1 Mol-% (bezogen auf das Epoxid) Dimethyldistearylammoniumchlorid (A3) und 2 Mol-% (bezogen auf das Epoxid) Natriumiodid versetzt. Die Reaktionsmischung wurde unter Stickstoff 510 min bei 200°C gerührt. Nach fraktionierter Destillation im Vakuum (0.08 bis 0.1 mbar, Sumpftemperatur 136 bis 153°C) wurde das Umlagerungsprodukt als farbloser Feststoff erhalten. Kenndaten des Produktes befinden sich in Tab.1.

**Tab.1**

| Kenndaten der Umlagerungsprodukte | | | | | | | | | | | | |
|---|---|---|---|---|---|---|---|---|---|---|---|---|
| Bsp. | E | A | A Mol-% | NaI Mol-% | T °C | t min | Ausb. % | COZ | OHZ | VZ | SZ | EpOZ Gew.-% |
| 1 | E1 | A1 | 5.0 | - | 180 | 165 | 76.9 | 62 | 32 | 189 | 0.6 | 0.1 |
| 2 | E1 | A1 | 1.0 | - | 180 | 585 | 79.5 | 68 | 33 | 188 | 1.0 | 0.1 |
| 3 | E1 | A2 | 1.0 | 5.0 | 180 | 195 | 77.7 | 62 | 27 | 185 | 1.2 | 0.1 |
| 4 | E1 | A2 | 1.0 | 1.0 | 210 | 420 | 78.1 | 57 | 33 | 188 | 0.6 | 0.1 |
| 5 | E1 | A3 | 1.1 | 4.3 | 180 | 585 | 72.9 | 66 | 19 | 188 | 1.2 | 0.3 |
| 6 | E1 | | | - | 180 | 240 | 78.6 | 63 | 28 | 184 | 1.3 | 0.1 |
| 7 | E2 | A2 | 2.0 | 1.0 | 180 | 420 | 97.0 | 98 | 23 | 3 | 0.2 | 0 |
| 8 | E3 | A1 | 1.0 | - | 200 | 480 | 94.0 | 29 | | 185 | 0.4 | 2.1 |
| 9 | E3 | A3 | 1.2 | 2.3 | 200 | 270 | 94.4 | 31 | | 182 | 0.5 | 1.9 |
| 10 | E4 | A1 | 0.64 | - | 200 | 540 | 96.0 | 21 | | 184 | 0.6 | 3.2 |
| 11 | E5 | A3 | 1.0 | 2.0 | 200 | 510 | 72.8 | 89 | | | | 0.3 |
| Legende: E = Edukt A = Tetrasubstituiertes Ammonium- bzw. Phosphoniumsalz Ausb = Ausbeute bezogen auf eingesetztes Epoxid COZ = Carbonylzahl OHZ = Hydroxylzahl | | | | | | | | | | | | |

## Patentansprüche

1. Verfahren zur Herstellung von Ketonverbindungen durch Umlagerung von Epoxiden, die mindestens eine Oxirangruppe und mindestens sechs Kohlenstoffatome enthalten, wobei mindestens einer der vier Substituenten der Oxirangruppe ein Wasserstoffatom darstellt, in Gegenwart von Iodidionen, **dadurch gekennzeichnet**, daß als Umlagerungskatalysatoren quartäre Ammonium- oder Phosphoniumsalze, gegebenenfalls im Gemisch mit Alkali- oder Erdalkaliiodiden, verwendet werden.

2. Verfahren nach Anspruch 1, dadurch gekennzeichnet, daß die Epoxide epoxidierte Monoolefine sind.

3. Verfahren nach Anspruch 1, dadurch gekennzeichnet, daß die Epoxide epoxidierte Alkyl-, Alkenyl-, Aralkyl- oder Phenylester von ungesättigten Fettsäuren sind.

4. Verfahren nach Anspruch 1, dadurch gekennzeichnet, daß die Epoxide epoxidierte Alkenylester von gesättigten Fettsäuren sind.

5. Verfahren nach Anspruch 1, dadurch gekennzeichnet, daß die Epoxide epoxidierte Alkenylether sind.

6. Verfahren nach einem der Ansprüche 1 bis 5, dadurch gekennzeichnet, daß als Umlagerungskatalystoren quartäre Phosphoniumiodide verwendet werden.

7. Verfahren nach einem der Ansprüche 1 bis 6, dadurch gekennzeichnet, daß als Umlagerungskatalysatoren quartäre Ammonium- oder Phosphoniumchloride oder -bromide im Gemisch mit Natriumiodid verwendet werden.

8. Verfahren nach einem der Ansprüche 1 bis 7, dadurch gekennzeichnet, daß die Konzentration der quartären Ammonium- oder Phosphoniumsalze 0.05 bis 10 Mol-% bezogen auf das Epoxid beträgt.

9. Verfahren nach einem der Ansprüche 1 bis 8, dadurch gekennzeichnet, daß die Konzentration des Alkali- oder Erdalkaliiodids 0.1 bis 8 Mol-% bezogen auf das Epoxid beträgt.

10. Verfahren nach einem der Ansprüche 1 bis 9, dadurch gekennzeichnet, daß das molare Verhältnis von quartärem Ammonium- oder Phosphoniumsalz zum Alkali- oder Erdalkaliiodid 2 : 1 bis 1 : 5 beträgt.

11. Verfahren nach einem der Ansprüche 1 bis 10, dadurch gekennzeichnet, daß die Umlagerung bei Temperaturen von 160 bis 230°C durchgeführt wird.

## Claims

1. A process for the production of ketone compounds by rearrangement of epoxides containing at least one oxirane group and at least six carbon atoms, at least one of the four substituents of the oxirane group being a hydrogen atom, in the presence of iodide ions, characterized in that quaternary ammonium or phosphonium salts, optionally in admixture with alkali metal or alkaline earth metal iodides, are used as the rearrangement catalysts.

2. A process as claimed in claim 1, characterized in that the epoxides are epoxidized monoolefins.

3. A process as claimed in claim 1, characterized in that the epoxides are epoxidized alkyl, alkenyl, aralkyl or phenyl esters of unsaturated fatty acids.

4. A process as claimed in claim 1, characterized in that the epoxides are epoxidized alkenyl esters of saturated fatty acids.

5. A process as claimed in claim 1, characterized in that the epoxides are epoxidized alkenyl ethers.

6. A process as claimed in any of claims 1 to 5, characterized in that quaternary phosphonium iodides are used as the rearrangement catalysts.

7. A process as claimed in any of claims 1 to 6, characterized in that quaternary ammonium or phosphonium chlorides or bromides are used in admixture with sodium iodide as the rearrangement catalysts.

8. A process as claimed in any of claims 1 to 7, characterized in that the concentration of the quaternary ammonium or phosphonium salts is from 0.05 to 10 mol-%, based on the epoxide.

9. A process as claimed in any of claims 1 to 8, characterized in that the concentration of the alkali metal or alkaline earth metal iodide is from 0.1 to 8 mol-%, based on the epoxide.

10. A process as claimed in any of claims 1 to 9, characterized in that the molar ratio of quaternary ammonium or phosphonium salt to the alkali metal or alkaline earth metal iodide is from 2:1 to 1:5.

11. A process as claimed in any of claims 1 to 10, characterized in that the rearrangement is carried out at temperatures of 160 to 230°C.

## Revendications

1. Procédé de préparation de composés de cétone par transposition d'époxydes, qui renferment au moins un groupe oxirane et au moins six atomes de carbone, un atome d'hydrogène constituant au moins un des quatres substituants du groupe oxirane, en présence d'ions d'iodure, caractérisé en ce que l'on utilise comme catalyseurs de transposition, des sels quaternaires de l'ammonium ou du phosphonium, le cas échéant en mélange avec des iodures de métaux alcalins ou alcalino-terreux.

2. Procédé selon la revendication 1, caractérisé en ce que les époxydes sont des monooléfines époxydées.

3. Procédé selon la revendication 1, caractérisé en ce que les époxydes sont des esters d'alkyle, d'alcényle, d'aralkyle ou de phényle époxydés d'acides gras insaturés.

4. Procédé selon la revendication 1, caractérisé en ce que les époxydes sont des esters d'alcényle époxydés d'acides gras saturés.

5. Procédé selon la revendication 1, caractérisé en ce que les époxydes sont des éthers d'alcényle époxydés.

6. Procédé selon une des revendications 1 à 5, caractérisé en ce que l'on utilise comme catalyseurs de transposition, des iodures quaternaires du phosphonium.

7. Procédé selon une des revendications 1 à 6, caractérisé en ce que l'on utilise comme catalyseurs de transposition, des chlorures ou des bromures quaternaires de l'ammonium ou du phosphonium, en mélange avec de l'iodure de sodium.

8. Procédé selon une des revendications 1 à 7, caractérisé en ce que la concentration des sels quaternaires de l'ammonium ou du phosphonium atteint 0,05 à 10 moles % par rapport à l'époxyde.

9. Procédé selon une des revendications 1 à 8, caractérisé en ce que la concentration de l'iodure de métal alcalin ou alcalino-terreux atteint 0,1 à 8 moles % par rapport à l'époxyde.

10. Procédé selon une des revendications 1 à 9, caractérisé en ce que le rapport molaire entre le sel quaternaire de l'ammonium ou du phosphonium et l'iodure de métal alcalin ou alcalino-terreux est compris entre 2:1 et 1:5.

11. Procédé selon une des revendications 1 à 10, caractérisé en ce que la transposition est réalisée à des températures comprises entre 160 et 230 °C.
